# EUROPEAN PATENT APPLICATION

(11) **EP 4 403 921 A1**
(43) Date of publication of application: **24.07.2024**
(21) Application number: 23209334.4
(22) Date of filing: 13.11.2023
(51) Int. Cl.: G01N 35/02, G01N 35/04

(54) **CONVEYING MECHANISM AND NUCLEIC ACID DETECTION APPARATUS**

(30) Priority: 19.01.2023 CN 202310141096
(71) Applicant: Shenzhen New Industries Biomedical Engineering Co., Ltd., Shenzhen, Guangdong 518122 (CN)
(72) Inventor: MU, Hailin, Shenzhen, 518122 (CN); XU, Fangyuan, Shenzhen, 518122 (CN); QIU, Zefeng, Shenzhen, 518122 (CN); CHENG, Yu, Shenzhen, 518122 (CN); HUANG, Ren, Shenzhen, 518122 (CN)
(74) Representative: Studio Torta S.p.A.

(57) **Abstract**

Provided in the present invention are a conveying mechanism and a nucleic acid detection apparatus. Specifically, the conveying mechanism includes: a frame; a movable plate, movably disposed on the frame in a horizontal direction, the movable plate having a material carrying position located above the frame and a material picking-up position protruding forward from the frame; and a first carrying table, movably disposed on the movable plate in a vertical direction. By applying the technical solutions of the present invention, the problems of complex structures and high production costs of nucleic acid detection apparatuses in the related art may be effectively solved.

## Description

### Cross-Reference to Related Application

The present invention claims priority to Chinese Patent Application No. 202310141096.2, filed to the China National Intellectual Property Administration on January, 19, 2023 and entitled "Conveying mechanism and Nucleic Acid Detection Apparatus", the disclosure of which is hereby incorporated by reference in its entirety.

### Technical Field

The present invention relates to the field of nucleic acid detection devices, and specifically, to a conveying mechanism and a nucleic acid detection apparatus.

### Background

A nucleic acid detection apparatus is configured to detect genetic materials and has important applications in the field of disease diagnosis.

A current fully-automatic nucleic acid detection apparatus generally includes a plurality of steps, such as nucleic acid extraction, amplification detection, etc. The nucleic acid detection apparatus in the related art generally transfers consumable materials for nucleic acid detection through a conveying mechanism.

However, due to different sizes and various types of involved consumable materials, when the conveying mechanism that may move in a horizontal direction transfers the consumable materials in cooperation with a material picking-up apparatus, the material picking-up apparatus is required to have an accurate positioning mechanism and a complex driving mechanism, resulting in complex structure and high production cost of the nucleic acid detection apparatus.

### Summary

The present invention is mainly intended to provide a conveying mechanism and a nucleic acid detection apparatus, to solve the problems of complex structures and high production costs of nucleic acid detection apparatuses in the related art.

In order to implement the above objective, an aspect of the present invention provides a conveying mechanism, which includes: a frame; a movable plate, movably disposed on the frame in a horizontal direction, the movable plate having a material carrying position located above the frame and a material picking-up position protruding forward from the frame; and a first carrying table, movably disposed on the movable plate in a vertical direction.

In some embodiments, the conveying mechanism further includes: a second carrying table, disposed on the movable plate and located on a circumferential outer side of the first carrying table. The first carrying table has a lifting position higher than the second carrying table and a descending position lower than the second carrying table.

In some embodiments, the first carrying table further has a flush position, which is flush with the second carrying table.

In some embodiments, the second carrying table is fixedly disposed on the movable plate.

In some embodiments, the first carrying table includes a first carrying plate located above the movable plate, and a first driving structure disposed between the first carrying plate and the movable plate; and the first driving structure is capable of driving the first carrying plate to move in the vertical direction.

In some embodiments, the second carrying table includes a second carrying plate located above the movable plate, and a support member fixedly connected with the movable plate and the second carrying plate; the first driving structure is disposed on the support member; the second carrying plate is provided with a mounting opening; and a projection of the second carrying plate on a horizontal plane surrounds a projection of the first carrying plate on the horizontal plane.

In some embodiments, the first driving structure includes: a driving motor, disposed on the support member, wherein a driving shaft of the driving motor extends in the horizontal direction; a driving gear, sleeving on the driving shaft of the driving motor and synchronously rotating with the driving shaft of the driving motor; and a rack structure. A first end of the rack structure is fixedly connected with the first carrying plate, and the rack structure meshes with the driving gear.

In some embodiments, the first carrying table further includes: a first guiding structure, including a sliding rail and a sliding block slidably disposed on the sliding rail. The sliding rail is disposed on the support member, and the sliding block is disposed on a second end of the rack structure.

In some embodiments, the first carrying table further includes: a first limit structure, disposed on the first carrying plate and being in limiting cooperation with a first consumable material.

In some embodiments, the first limit structure includes a plurality of limit columns disposed on the first carrying table.

In some embodiments, the first limit structure further includes a plurality of limit convex ribs disposed on the first carrying table; and the plurality of limit convex ribs are arranged at intervals on a circumferential edge of the first carrying plate.

In some embodiments, the plurality of limit convex ribs are located on a circumferential outer side of the plurality of limit columns; and the limit convex ribs and the limit columns are arranged at intervals.

In some embodiments, an upper surface of each limit convex rib is an inclined plane; and the inclined plane gradually inclines downwards in a direction from an outer side to an inner side of the first carrying table.

In some embodiments, the second carrying table further includes: a second limit structure, disposed on the second carrying plate and being in limiting cooperation with a second consumable material. When the first carrying table is located at the descending position, the second limit structure is lower than the first carrying plate.

In some embodiments, a front end of the movable plate protrudes forwards from a front end of the first carrying table, and protrudes forwards from a front end of the second carrying table.

In some embodiments, the conveying mechanism further includes: a second guiding structure, disposed between the frame and the movable plate, wherein the movable plate is movably disposed on the frame through the second guiding structure; and a second driving structure, disposed on the frame and being in driving connection with the movable plate. The second driving structure is capable of driving the movable plate to move between the material picking-up position and the material carrying position.

Another aspect of the present invention provides a nucleic acid detection apparatus, which includes: a base, including a first area and a second area, which are disposed adjacent to each other; a separation structure, disposed between the first area and the second area and configured to separate the first area and the second area, wherein the separation structure includes a rotary door; and the rotary door has an open position connecting the first area and the second area, and a close position separating the first area and the second area; and a conveying mechanism. The conveying mechanism is the above conveying mechanism; the frame of the conveying mechanism is disposed in the second area; when the movable plate of the conveying mechanism is located at the material carrying position, the movable plate is located in the second area; when the movable plate is located at the material picking-up position, the movable plate is located in the first area; and when the movable plate moves from the material carrying position to the material picking-up position, the movable plate is capable of being in pushing cooperation with the rotary door.

By applying the technical solutions of the present invention, the conveying mechanism includes the frame and the movable plate movably disposed on the frame. The movable plate may carry a consumable material. When the conveying mechanism is assembled, the frame may be fixed in an area. Since the movable plate is capable of moving in the horizontal direction and has the material carrying position and the material picking-up position, the movable plate may transport the consumable material during moving. In addition, the movable plate of the conveying mechanism is also provided with the first carrying table. When the conveying mechanism transports consumable materials with different heights, the height of the first carrying table may be adjusted, so as to decrease a height difference of different consumable materials at a specific position. Through such arrangement, the material picking-up difficulty of a material picking-up apparatus may be reduced, such that the design difficulty and processing costs of the material picking-up apparatus are reduced, thereby facilitating reduction of the processing cost of the nucleic acid detection apparatus.

### Brief Description of the Drawings

The drawings, which form a part of this application, are used to provide a further understanding of the present invention. The exemplary embodiments of the present invention and the description thereof are used to explain the present invention, but do not constitute improper limitations to the present invention. In the drawings:
Fig. 1 is a schematic diagram of a three-dimensional structure from a first angle of an embodiment of a conveying mechanism according to the present invention.
Fig. 2 is a schematic diagram of a three-dimensional structure from a second angle of the conveying mechanism of Fig. 1, wherein Fig. 2 is a schematic structural diagram of a first carrying table being at a lifting position and carrying an amplification plate body.
Fig. 3 is a schematic diagram of a three-dimensional structure from a third angle of the conveying mechanism of Fig. 1, wherein Fig. 3 is a schematic structural diagram of the first carrying table being at the lifting position and carrying a cover plate.
Fig. 4 is a schematic diagram of a three-dimensional structure from a fourth angle of the conveying mechanism of Fig. 1, wherein Fig. 4 is a schematic structural diagram of the first carrying table being at a descending position and carrying a second consumable material.
Fig. 5 is a schematic diagram of a three-dimensional structure from a fifth angle of the conveying mechanism of Fig. 1.
Fig. 6 is a schematic diagram of a partial structure of the conveying mechanism of Fig. 3.
Fig. 7 is a schematic diagram of a partial structure of an embodiment of a nucleic acid detection apparatus according to the present invention.

The above accompanying drawings include the following reference numerals:
10. Frame; 20. Movable plate; 30. First carrying table; 31. First carrying plate; 32. First driving structure; 321. Driving motor; 322. Driving gear; 323. Rack structure; 33. First limit structure; 331. Limit column; 332. Limit convex rib; 3321. Inclined plane; 40. Second carrying table; 41. Second carrying plate; 411. Mounting opening; 42. Support member; 43. Second limit structure; 50. First guiding structure; 51. Sliding rail; 52. Sliding block; 60. Second guiding structure; 70. Second driving structure; 90. Separation structure; 91. Rotary door; 100. Conveying mechanism; 110. First consumable material; 111. Cover plate; 112. Amplification plate body; 120. Second consumable material.

### Detailed Description of the Embodiments

The technical solutions in the embodiments of the present invention will be clearly and completely described below in combination with the drawings in the embodiments of the present invention. It is apparent that the described embodiments are only part of the embodiments of the present invention, not all the embodiments. The following description of at least one exemplary embodiment is merely illustrative in nature and is never intended to limit the present invention and application or use thereof in any way. Based on the embodiments in the present invention, all other embodiments obtained by those of ordinary skill in the art without creative work shall fall within the protection scope of the present invention.

It is to be noted that, terms used herein are intended to describe specific implementations only and are not intended to limit exemplary embodiments according to the present application. As used herein, unless the context clearly indicates otherwise, a singular form is also intended to include a plural form. In addition, it is further understood that when the terms "including" and/or "comprising" are used in this specification, the terms indicate the presence of features, steps, operations, devices, components, and/or a combination thereof.

Unless specifically stated otherwise, the relative arrangement, numerical expressions, and numerical values of the components and steps set forth in these embodiments do not limit the scope of the present invention. In addition, it is to be understood that the dimension of each portion shown in the drawings is not drawn to actual scale for ease of description. Techniques, methods, and devices known to those of ordinary skill in the relevant art may not be discussed in detail, but where appropriate, such techniques, methods, and devices should be considered as a part of the authorized specification. In all examples shown and discussed herein, any specific value should be construed as illustrative merely and not as limitations. Accordingly, other examples of the exemplary embodiments may have different values. It is to be noted that similar numbers and letters indicate similar items in the following drawings, so once a certain item is defined in one drawing, no further discussions are required for same in the subsequent drawings.

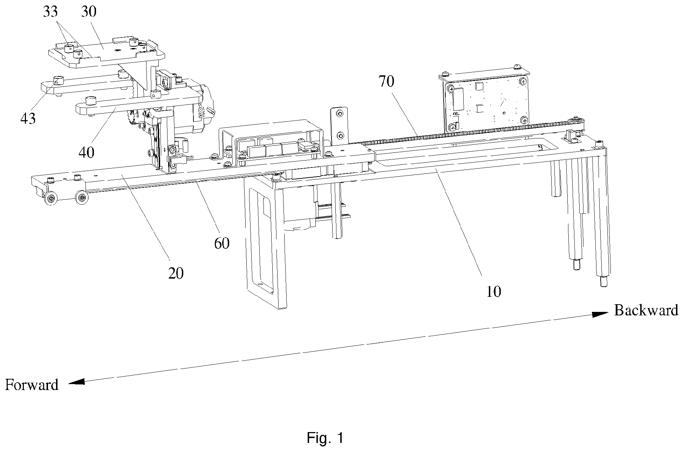

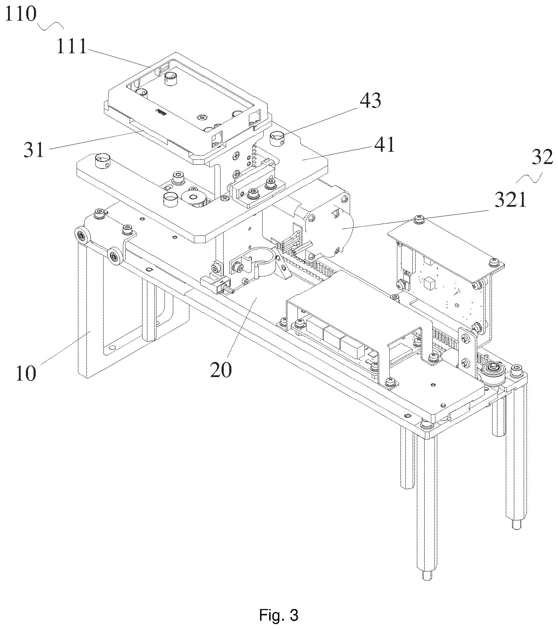

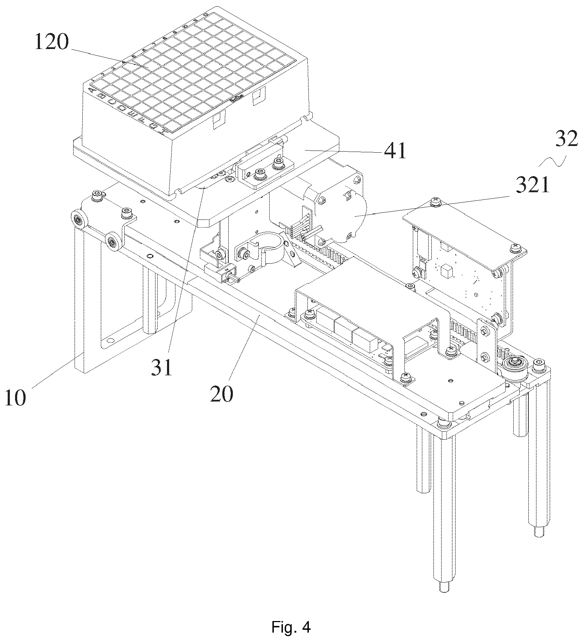

As shown in Fig. 1 to Fig. 5, the conveying mechanism of this embodiment includes a frame 10, a movable plate 20 and a first carrying table 30. The movable plate 20 is movably disposed on the frame 10 in a horizontal direction. The movable plate 20 has a material carrying position located above the frame 10 and a material picking-up position protruding forward from the frame 10. The first carrying table 30 is movably disposed on the movable plate 20 in a vertical direction.

By applying the technical solutions of this embodiment, the conveying mechanism includes the frame 10 and the movable plate 20 movably disposed on the frame 10. When the conveying mechanism is assembled, the frame 10 may be fixed in an area. Since the movable plate 20 is capable of moving in the horizontal direction and has the material carrying position and the material picking-up position, the movable plate 20 may transport the consumable material during moving. In addition, the movable plate 20 of the conveying mechanism is also provided with the first carrying table 30, and the first carrying table 30 may carry the consumable material. When the conveying mechanism transports the consumable materials with different heights, the height of the first carrying table 30 may be adjusted, so as to decrease a height difference of different consumable materials at a specific position (which is a position that the consumable materials need to be transported, when the consumable materials are transported to the specific position, a material picking-up apparatus may be in grabbing cooperation with the consumable materials). Through such arrangement, the material picking-up difficulty of the material picking-up apparatus may be reduced, such that the design difficulty and processing costs of the material picking-up apparatus are reduced, thereby facilitating reduction of the processing cost of the nucleic acid detection apparatus.

It is to be noted that, the above "horizontal direction" refers to a forward-backward direction shown in Fig. 1.

As shown in Fig. 1 to Fig. 5, in this embodiment, the conveying mechanism further includes: a second carrying table 40, disposed on the movable plate 20 and located on a circumferential outer side of the first carrying table 30. The first carrying table 30 has a lifting position higher than the second carrying table 40 and a descending position lower than the second carrying table 40. In the above structure, since the conveying mechanism needs to transport different consumable materials, when an area occupied by the consumable materials that need to be transported is relatively small, the consumable materials may be placed on the first carrying table 30. When the area occupied by the consumable materials that need to be transported is relatively large, the first carrying table 30 may be moved to the descending position, such that the consumable materials can be placed on the second carrying table 40. Through the above structure, the conveying mechanism may select the appropriate carrying table for transportation according to the size of the consumable material that needs to be transported, such that stability during consumable material transportation is improved.

The feature that "the second carrying table 40 is disposed on the movable plate 20 and located on the circumferential outer side of the first carrying table 30" means that the second carrying table 40 may completely surround the first carrying table 30, or may partially surround the first carrying table 30.

It is to be noted that, in this embodiment, the consumable materials include an amplification plate and an extraction plate. An area occupied by the amplification plate is smaller, such that the amplification plate may be transported by the first carrying table 30. A circumferential edge of the first carrying table 30 is located outside a circumferential edge of the amplification plate, the probability that the edge of the amplification plate is touched during transportation may be reduced, such that the amplification plate may be stably placed at a predetermined position, and the material picking-up apparatus may successfully pick up the amplification plate. An area occupied by the extraction plate is larger, such that the extraction plate may be transported by the second carrying table 40. A circumferential edge of the second carrying table 40 is located outside a circumferential edge of the extraction plate, the probability that the edge of the extraction plate is touched during transportation may be reduced, such that the extraction plate may be stably placed at a predetermined position, and the material picking-up apparatus may successfully pick up the extraction plate.

It is further to be noted that, in other embodiments not shown in the figures, the first carrying table 30 further has a flush position, which is flush with the second carrying table 40.

As shown in Fig. 1 to Fig. 5, in this embodiment, the second carrying table 40 is fixedly disposed on the movable plate 20. In the above structure, the second carrying table 40 is configured to transport the extraction plate, and the height of the extraction plate is greater than the amplification plate. Therefore, the second carrying table 40 may be fixedly disposed, and the first carrying table 30 is movably disposed in the vertical direction. By increasing the height of the first carrying table 30 to reduce the height difference of different consumable materials at the specific position, such that the material picking-up apparatus may conveniently pick up the materials. Through the above arrangement, the production cost of the second carrying table 40 is reduced, such that the production cost of the conveying mechanism is reduced, thereby reducing the production cost of the nucleic acid detection apparatus.

Definitely, in other embodiments not shown in the figures, the second carrying table 40 may also be movably disposed on the movable plate 20 in the vertical direction. Through the above arrangement, the flexibility of the second carrying table 40 may be improved, such that the type of the consumable material which transported by the conveying mechanism is increased, thereby improving the universality of the conveying mechanism.

As shown in Fig. 1 to Fig. 5, in this embodiment, the first carrying table 30 includes a first carrying plate 31 located above the movable plate 20, and a first driving structure 32 disposed between the first carrying plate 31 and the movable plate 20; and the first driving structure 32 is capable of driving the first carrying plate 31 to move in the vertical direction. In the above structure, an automation level of the conveying mechanism may be improved by using the first driving structure 32 to drive the first carrying plate 31 to lift up and down, such that the transportation efficiency of the consumable material is improved.

As shown in Fig. 1 to Fig. 5, in this embodiment, the second carrying table 40 includes a second carrying plate 41 located above the movable plate 20, and a support member 42 fixedly connected with the movable plate 20 and the second carrying plate 41. The first driving structure 32 is disposed on the support member 42; the second carrying plate 41 is provided with a mounting opening 411; and a projection of the second carrying plate 41 on a horizontal plane surrounds a projection of the first carrying plate 31 on the horizontal plane. In the above structure, the second carrying plate 41 is supported by the support member 42, such that the second carrying plate 41 may be stably connected. The first driving structure 32 is mounted on the support member 42; and the first carrying plate 31 may extend into the mounting opening 411. Through the above structure, the arrangement of the first carrying table 30 and the second carrying table 40 is compact in layout, such that the space occupied by the conveying mechanism is reduced, so as to cause the nucleic acid detection apparatus to be compact in design.

It is to be noted that, the second carrying plate 41 is a U-shaped structure.

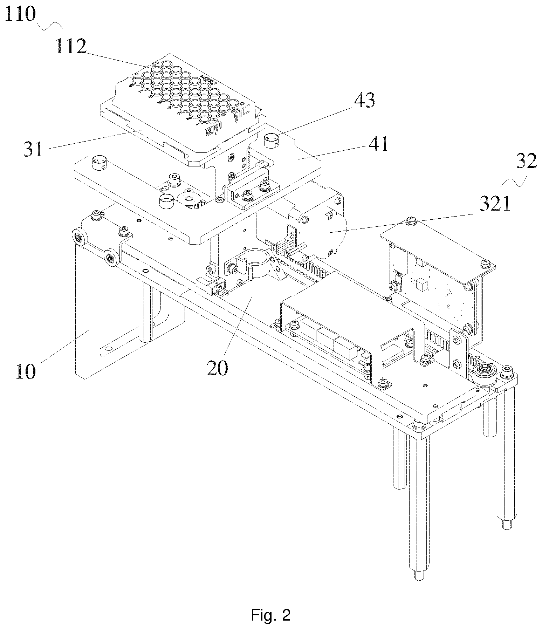

As shown in Fig. 2 to Fig. 5, in this embodiment, the first driving structure 32 includes a driving motor 321, a driving gear 322 and a rack structure 323. The driving motor 321 is disposed on the support member 42, and a driving shaft of the driving motor 321 extends in the horizontal direction. The driving gear 322 sleeves on the driving shaft of the driving motor 321 and synchronously rotates with the driving shaft of the driving motor 321. A first end of the rack structure 323 is fixedly connected with the first carrying plate 31, and the rack structure 323 meshes with the driving gear 322. In the above structure, the driving motor 321 drives, through the driving gear 322, the rack structure 323 to lift up and down, so as to drive the first carrying plate 31 to move vertically. The above structure is simple, low in production cost, and high in driving stability.

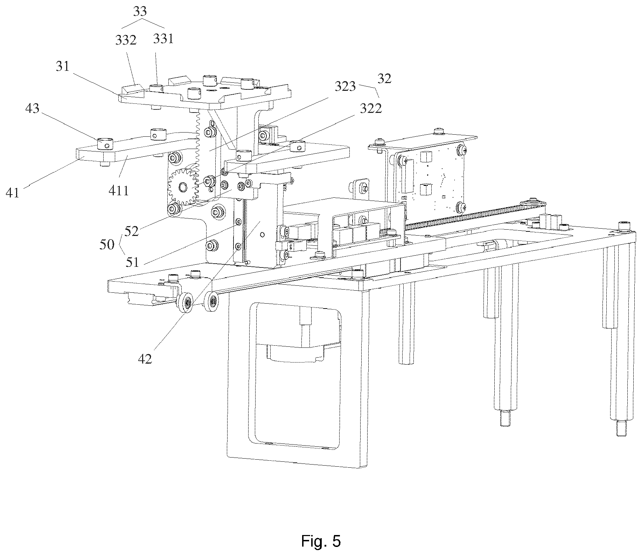

As shown in Fig. 5, in this embodiment, the first carrying table 30 further includes: a first guiding structure 50, including a sliding rail 51 and a sliding block 52 slidably disposed on the sliding rail 51. The sliding rail 51 is disposed on the support member 42, and the sliding block 52 is disposed on a second end of the rack structure 323. In the above structure, the first guiding structure 50 may further improve the moving stability of the rack structure 323, such that the moving stability of the first carrying plate 31 is guaranteed.

As shown in Fig. 1, in this embodiment, the first carrying table 30 further includes: a first limit structure 33, disposed on the first carrying plate 31 and being in limiting cooperating with a first consumable material 110. In the above structure, the first consumable material 110 is the amplification plate. The first limit structure 33 is in limiting cooperation with the amplification plate, such that the stability of the amplification plate during transportation is guaranteed.

Specifically, as shown in Fig. 2, Fig. 3 and Fig. 5, in this embodiment, the first limit structure 33 includes a plurality of limit columns 331 disposed on the first carrying table 30. Specifically, the amplification plate is a split structure, including an amplification plate body 112 and a cover plate 111 disposed on the amplification plate body 112. The amplification plate body 112 includes a top plate and a side plate disposed in a circumferential direction of the top plate. The top plate is provided with a plurality of downward through holes, and a cone-shaped pipe is mounted at a lower part of each through hole. The plurality of limit columns 331 are located in a frame enclosed by the side plate, and an upper surface of each limit column 331 is provided with a cone-shaped groove; and the cone-shaped groove is in limiting cooperation with the cone-shaped pipe, so as to limit the amplification plate.

As shown in Fig. 2, Fig. 3 and Fig. 5, in this embodiment, the first limit structure 33 further includes a plurality of limit convex ribs 332 disposed on the first carrying table 30; and the plurality of limit convex ribs 332 are arranged at intervals on a circumferential edge of the first carrying plate 31. In the above structure, the plurality of limit convex ribs 332 may limit the circumferential edge of the amplification plate, such that the stability of the amplification plate during transportation may also be guaranteed.

As shown in Fig. 2, Fig. 3 and Fig. 5, in this embodiment, the plurality of limit convex ribs 332 are located on a circumferential outer side of the plurality of limit columns 331; and the limit convex ribs 332 and the limit columns 331 are arranged at intervals. In the above structure, the plurality of limit columns 331 are configured to be in limiting cooperation with the amplification plate body 112; and the plurality of limit convex ribs 332 are configured to be in limiting cooperation with the cover plate 111. The plurality of limit convex ribs 332 and the plurality of limit columns 331 are arranged at intervals, such that when the first carrying plate 31 carries the amplification plate body 112, the limit convex ribs 332 do not interfere with the amplification plate body 112. Correspondingly, when the first carrying plate 31 carries the cover plate 111, the limit columns 331 do not interfere with the cover plate 111.

The amplification plate body 112 and the cover plate 111 need to be separately transported. The plurality of limit columns 331 are configured to be in limiting cooperation with the amplification plate body 112; and the plurality of limit convex ribs 332 are configured to be in limiting cooperation with the cover plate 111.

It is to be noted that, when the amplification plate body 112 is placed on the first carrying table and is in limiting cooperation with the plurality of limit columns 331, the plurality of limit convex ribs 332 are located on the circumferential outer side of the amplification plate body 112.

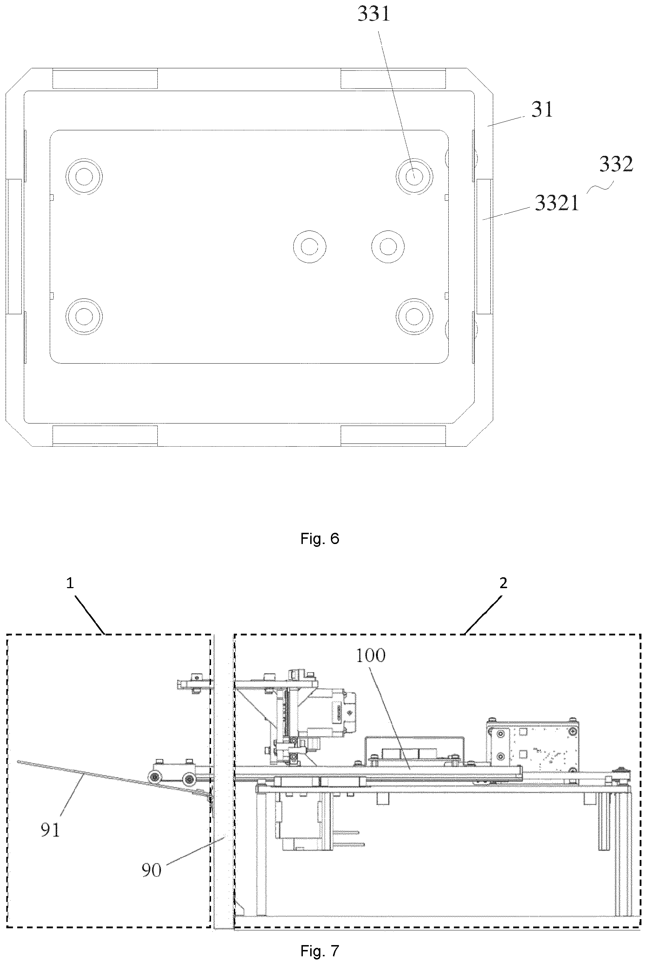

Specifically, as shown in Fig. 5 and Fig. 6, in this embodiment, an upper surface of each limit convex rib 332 is an inclined plane 3321; and the inclined plane 3321 gradually inclines downwards in a direction from an outer side to an inner side of the first carrying table 30. In the above structure, the inclined plane 3321 may play a role in guiding the cover plate 111 of the amplification plate. When an edge of the cover plate 111 is lapped on the inclined plane 3321, the cover plate 111 may slide down under the guidance of the inclined plane 3321, and then is limited by an inner wall of the limit convex rib 332, such that the cover plate 111 may be stably placed on the first carrying table 30.

As shown in Fig. 1, Fig. 4 and Fig. 5, in this embodiment, the second carrying table 40 further includes: a second limit structure 43, disposed on the second carrying plate 41 and being in limiting cooperation with a second consumable material 120. When the first carrying table 30 is located at the descending position, the first limit structure 33 is lower than the second carrying plate 41. In the above structure, the second consumable material 120 is the extraction plate. The extraction plate is provided with a cooperating structure cooperating with the second limit structure 43. Specifically, the extraction plate is provided with a plurality of cone-shaped pipes extending downwardly. The second limit structure 43 is a plurality of limit columns, and an upper surface of each limit column is provided with a cone-shaped groove; and the cone-shaped groove is in limiting cooperation with the cone-shaped pipe, so as to limit the extraction plate. In addition, it is further to be noted that, in order to prevent the first limit structure 33 on the first carrying table 30 from interfering with the extraction plate when the extraction plate is placed on the second carrying table 40, when the first carrying table is located at the descending position, the first limit structure 33 needs to be lower than the second carrying plate 41.

As shown in Fig. 1, in this embodiment, the conveying mechanism further includes a second guiding structure 60. The second guiding structure 60 is disposed between the frame 10 and the movable plate 20, wherein the movable plate 20 is movably disposed on the frame 10 through the second guiding structure 60. The above structure may guarantee the stability of the movable plate 20 when moving between the material carrying position and the material picking-up position, such that the probability of moving of the consumable material during transportation is reduced.

As shown in Fig. 1, in this embodiment, the conveying mechanism further includes a second driving structure 70. The second driving structure 70 is disposed on the frame 10 and being in driving connection with the movable plate 20, wherein the second driving structure 70 is capable of driving the movable plate 20 to move between the material picking-up position and the material carrying position. In the above structure, the movable plate 20 may be driven to move between the material picking-up position and the material carrying position by using a control program to control the second driving structure 70, such that the automation level of the conveying mechanism is improved.

As shown in Fig. 7, the nucleic acid detection apparatus of this embodiment includes a base, a separation structure 90 and a conveying mechanism 100. The base includes a first area 1 and a second area 2, which are disposed adjacent to each other. The separation structure 90 is disposed between the first area 1 and the second area 2 and configured to separate the first area 1 and the second area 2. The separation structure 90 includes a rotary door 91; and the rotary door 91 has an open position connecting the first area 1 and the second area 2, and a close position separating the first area 1 and the second area 2. The conveying mechanism 100 is the above conveying mechanism; the frame 10 of the conveying mechanism 100 is disposed in the second area 2; when the movable plate 20 of the conveying mechanism 100 is located at the material carrying position, the movable plate 20 is located in the second area 2; when the movable plate 20 is located at the material picking-up position, the movable plate 20 is located in the first area 1; and when the movable plate 20 moves from the material carrying position to the material picking-up position, the movable plate 20 is capable of being in pushing cooperation with the rotary door 91. In the above structure, the arrangement of the separation structure 90 facilitates physically separating the first area 1 and the second area 2. In addition, the separation structure 90 includes the rotary door 91. When the conveying mechanism 100 moves from the material carrying position to the material picking-up position, the movable plate 20 of the conveying mechanism 100 is capable of being in pushing cooperation with the rotary door 91. Therefore, the smooth moving of the conveying mechanism 100 across areas is guaranteed.

It is to be noted that, the first area 1 is a reagent configuration area; the reagent configuration area is configured to prepare sample extraction reagents and prepare sample amplification reagents; and there is no detection sample in the reagent configuration area, thus belonging to a low pollution area. The second area 2 is a sample extraction area; the sample extraction area is configured to place the detection samples and the sample extraction reagents; and extraction of the detection samples is completed in this area, such that this area belongs to a moderate pollution area.

Specifically, as shown in Fig. 7, in this embodiment, a front end of the movable plate 20 protrudes forwards from a front end of the first carrying table 30, and protrudes forwards from a front end of the second carrying table 40. Through the above structure, the movable plate 20 is capable of being in contact with the rotary door 91 prior to the first carrying table 30 and the second carrying table 40, such that the consumable material carried on the first carrying table 30 or the second carrying table 40 is prevented from touching the rotary door 91 prior to the movable plate 20, to change the placing position of the consumable material, thereby causing the material picking-up apparatus to hardly accurately grab the consumable material.

In the description of the present invention, it is to be understood that, terms such as "front, rear, up, down, left or right", "transverse, longitudinal, vertical, or horizontal", "top or bottom", and the like are usually based on the orientation or positional relationships shown in the drawings and are used only to facilitate and simplify the description of the present invention. In the absence of any indication to the contrary, these orientation words do not indicate and imply that the device or component referred to must have a particular orientation or be constructed and operated in a particular orientation, and therefore cannot be construed as limiting the scope of protection of the present invention. The orientation word "inside or outside" refers to the inside and outside relative to the contours of the components themselves.

For ease of description, spatially relative terms such as "on...", "above...", "on an upper surface of...", "upper", and the like may be used here to describe the spatial position relationship between a device or feature and other devices or features as shown in the figure. It is to be understood that, the spatially relative terms are intended to cover different orientations in use or operation other than the orientation of the device described in the figure. For example, if the devices in the drawings are inverted, the devices described as "above" or "on" other devices or configurations will later be positioned as "below" or "under" other devices or configurations. Therefore, the exemplary term "above..." may include both orientations of "above..." and "below...". The device may also be positioned in other different ways (rotating 90 degrees or in other orientations), and the spatially relative descriptions used here are explained accordingly.

In addition, it is to be noted that, the use of words such as "first" and "second" to limit the parts is only to facilitate the distinguishing of corresponding parts, if not otherwise stated, the above words do not have a special meaning, and therefore cannot be understood as a limitation of the scope of protection of the present invention.

The above are only the preferred embodiments of the present invention and are not intended to limit the present invention. For those skilled in the art, the present invention may have various modifications and variations. Any modifications, equivalent replacements, improvements and the like made within the spirit and principle of the present invention all fall within the scope of protection of the present invention.

## Claims

1. A conveying mechanism, comprising:
a frame (10);
a movable plate (20), movably disposed on the frame (10) in a horizontal direction, wherein the movable plate (20) has a material carrying position located above the frame (10) and a material picking-up position protruding forward from the frame (10); and
a first carrying table (30), movably disposed on the movable plate (20) in a vertical direction.

2. The conveying mechanism according to claim 1, further comprising:
a second carrying table (40), disposed on the movable plate (20) and located on a circumferential outer side of the first carrying table (30), wherein the first carrying table (30) has a lifting position higher than the second carrying table (40) and a descending position lower than the second carrying table (40).

3. The conveying mechanism according to claim 2, wherein the first carrying table (30) further has a flush position, which is flush with the second carrying table (40).

4. The conveying mechanism according to claim 2, wherein
the second carrying table (40) is fixedly disposed on the movable plate (20).

5. The conveying mechanism according to any one of claims 2 to 4, wherein the first carrying table (30) comprises a first carrying plate (31) located above the movable plate (20), and a first driving structure (32) disposed between the first carrying plate (31) and the movable plate (20); and the first driving structure (32) is capable of driving the first carrying plate (31) to move in the vertical direction.

6. The conveying mechanism according to claim 5, wherein the second carrying table (40) comprises a second carrying plate (41) located above the movable plate (20), and a support member (42) fixedly connected with the movable plate (20) and the second carrying plate (41); the first driving structure (32) is disposed on the support member (42); the second carrying plate (41) is provided with a mounting opening (411); and a projection of the second carrying plate (41) on a horizontal plane surrounds a projection of the first carrying plate (31) on the horizontal plane.

7. The conveying mechanism according to claim 6, wherein the first driving structure (32) comprises:
a driving motor (321), disposed on the support member (42), wherein a driving shaft of the driving motor (321) extends in the horizontal direction;
a driving gear (322), sleeving on the driving shaft of the driving motor (321) and synchronously rotating with the driving shaft of the driving motor (321); and
a rack structure (323), wherein a first end of the rack structure (323) is fixedly connected with the first carrying plate (31), and the rack structure (323) meshes with the driving gear (322).

8. The conveying mechanism according to claim 7, wherein the first carrying table (30) further comprises:
a first guiding structure (50), comprising a sliding rail (51) and a sliding block (52) slidably disposed on the sliding rail (51), wherein the sliding rail (51) is disposed on the support member (42), and the sliding block (52) is disposed on a second end of the rack structure (323).

9. The conveying mechanism according to claim 6, wherein the first carrying table (30) further comprises:
a first limit structure (33), disposed on the first carrying plate (31) and being in limiting cooperation with a first consumable material (110),
wherein the first limit structure (33) comprises a plurality of limit columns (331) disposed on the first carrying plate (31).

10. The conveying mechanism according to claim 9, wherein the first limit structure (33) further comprises a plurality of limit convex ribs (332) disposed on the first carrying table (30); and the plurality of limit convex ribs (332) are arranged at intervals on a circumferential edge of the first carrying plate (31).

11. The conveying mechanism according to claim 10, wherein the plurality of limit convex ribs (332) are located on a circumferential outer side of the plurality of limit columns (331); and the limit convex ribs (332) and the limit columns (331) are arranged at intervals, and/or, an upper surface of each limit convex rib (332) is an inclined plane (3321); and the inclined plane (3321) gradually inclines downwards in a direction from an outer side to an inner side of the first carrying table (30).

12. The conveying mechanism according to claim 9, wherein the second carrying table (40) further comprises:
a second limit structure (43), disposed on the second carrying plate (41) and being in limiting cooperation with a second consumable material (120), wherein when the first carrying table (30) is located at the descending position, the first limit structure (33) is lower than the second carrying plate (41).

13. The conveying mechanism according to claim 2, wherein a front end of the movable plate (20) protrudes forwards from a front end of the first carrying table (30), and protrudes forwards from a front end of the second carrying table (40).

14. The conveying mechanism according to claim 1, further comprising:
a second guiding structure (60), disposed between the frame (10) and the movable plate (20), wherein the movable plate (20) is movably disposed on the frame (10) through the second guiding structure (60); and
a second driving structure (70), disposed on the frame (10) and being in driving connection with the movable plate (20), wherein the second driving structure (70) is capable of driving the movable plate (20) to move between the material picking-up position and the material carrying position.

15. A nucleic acid detection apparatus, comprising:
a base, comprising a first area (1) and a second area (2), which are disposed adjacent to each other;
a separation structure (90), disposed between the first area (1) and the second area (2) and configured to separate the first area (1) and the second area (2), wherein the separation structure (90) comprises a rotary door (91); and the rotary door (91) has an open position connecting the first area (1) and the second area (2), and a close position separating the first area (1) and the second area (2); and
a conveying mechanism (100), wherein the conveying mechanism (100) is the conveying mechanism according to any one of claims 1 to 14; the frame (10) of the conveying mechanism (100) is disposed in the second area (2); when the movable plate (20) of the conveying mechanism (100) is located at the material carrying position, the movable plate (20) is located in the second area (2); when the movable plate (20) is located at the material picking-up position, the movable plate (20) is located in the first area (1); and when the movable plate (20) moves from the material carrying position to the material picking-up position, the movable plate (20) is capable of being in pushing cooperating with the rotary door (91).
